(19) Europäisches Patentamt
European Patent Office
Office européen des brevets

(11) Veröffentlichungsnummer: **0 372 667 A1**

# (12) EUROPÄISCHE PATENTANMELDUNG

(21) Anmeldenummer: **89250096.8**

(22) Anmeldetag: **27.11.89**

(51) Int. Cl.⁵: **C07D 333/20, C07D 333/54, A61K 31/38**

(30) Priorität: **29.11.88 DE 3840802**

(43) Veröffentlichungstag der Anmeldung: **13.06.90 Patentblatt 90/24**

(84) Benannte Vertragsstaaten: **AT BE CH DE ES FR GB IT LI NL SE**

(71) Anmelder: **SCHERING AKTIENGESELLSCHAFT**
**Berlin und Bergkamen**
**Müllerstrasse 170/78**
**D-1000 Berlin 65(DE)**

(72) Erfinder: **Albrecht, Rudolf, Dr.**
**Wunsiedeler Weg 37**
**D-1000 Berlin 46(DE)**
Erfinder: **Schöllkopf, Klaus, Dr.**
**Kurstrasse 6**
**D-1000 Berlin 38(DE)**
Erfinder: **Schröder, Gertrud, Dr.**
**Theodor-Francke-Strasse 3**
**D-1000 Berlin 42(DE)**
Erfinder: **Schulz, Bernd-Günter, Dr.**
**Kurfürstenstrasse 14**
**D-1000 Berlin 28(DE)**

## (54) 2-Hydroxymethylthiophen-Derivate, Verfahren zu ihrer Herstellung und ihre Verwendung als Arzneimittel.

(57) Die Erfindung betrifft 2-Hydroxymethylthiophen-Derivate der Formel I

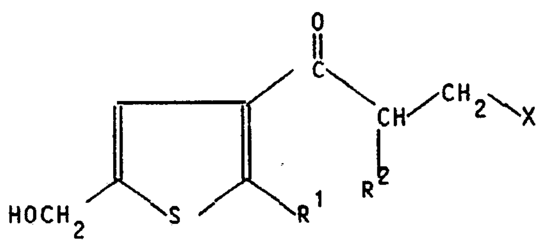

(I),

worin
$R^1$ und $R^2$ Wasserstoff oder gemeinsam eine Ethylengruppe,
$R^3$ und $R^4$ Wasserstoff oder gemeinsam eine Ethylengruppe und
X die Gruppe

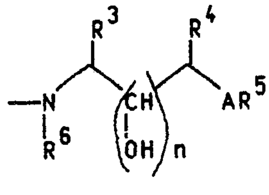

mit $R^5$ in der Bedeutung eines $C_1$-$C_4$-Alkyls oder eines gegebenenfalls substituierten Phenylrestes, mit n in der Bedeutung 1 oder 0, mit A in der Bedeutung eines Sauerstoffatoms oder einer direkten Bindung oder,

EP 0 372 667 A1

wenn n = 0 und $R^3$ und $R^4$ = H sind, mit einer gemeinsamen $-CH_2-CH_2-CH-$-Gruppe für $R^6$ und A darstellen, deren Säureadditionssalze, Verfahren zu ihrer Herstellung sowie deren pharmazeutische Verwendung.

# 2-HYDROXYMETHYLTHIOPHEN-DERIVATE, VERFAHREN ZU IHRER HERSTELLUNG UND IHRE VERWENDUNG ALS ARZNEIMITTEL

Die vorliegende Erfindung betrifft 2-Hydroxymethylthiophen-Derivate, Verfahren zu ihrer Herstellung und ihre Verwendung als Arzneimittel.

Aus EP 127143 sind substituierte 5-Aminomethyl-4,5,6,7-tetrahydro-2-methyl-benzothiophen-4-one bekannt, über deren blutdrucksenkende Wirkung J.R. McCarthy et al. in J. Med. Chem. 28, 1142 (1985) berichten.

Es wurde nun gefunden, daß 2-Hydroxymethylthiophen-Derivate, die anstelle der 2-Methylgruppe eine 2-Hydroxymethylgruppe am Thiophenring tragen, ein verbessertes pharmakologisches Wirkprofil aufweisen, indem bei diesen Verbindungen die Blutdrucksenkung nicht von einer Steigerung der Herzfrequenz, die bei einem Antihypertensivium unerwünscht ist, begleitet wird.

Die Erfindung betrifft somit 2-Hydroxymethylthiophen-Derivate der Formel I

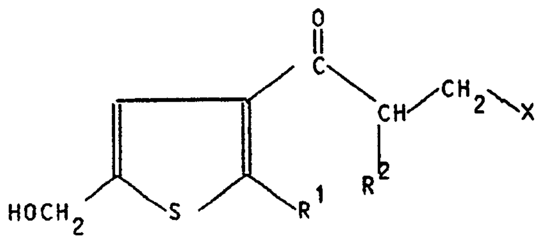

(I),

worin
$R^1$ und $R^2$ Wasserstoff oder gemeinsam eine Ethylengruppe,
$R^3$ und $R^4$ Wasserstoff oder gemeinsam eine Ethylengruppe und
X die Gruppe

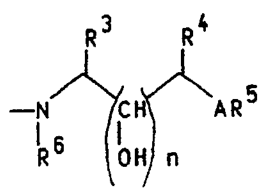

mit $R^5$ in der Bedeutung eines $C_1$-$C_4$-Alkyls oder eines gegebenenfalls substituierten Phenylrestes, mit n in der Bedeutung 1 oder 0, mit A in der Bedeutung eines Sauerstoffatoms oder einer direkten Bindung oder, wenn n = 0 und $R^3$ und $R^4$ = H sind, mit einer gemeinsamen $-CH_2-CH_2-CH-$-Gruppe

für $R^6$ und A darstellen sowie deren Säureadditionssalze.

Mit $C_1$-$C_4$-Alkylresten von $R^5$ sind gerad- und verzweigtkettige Alkylreste wie z.B. Methyl, Ethyl, n-Propyl, Isopropyl, n-Butyl, Isobutyl, sek.-Butyl und tert.-Butyl gemeint.

Unter Substitution des gegebenenfalls substituierten Phenylrestes in der Definition von $R^5$ sind zu verstehen: Eine Hydroxygruppe oder mehrere Hydroxygruppen, eine $C_1$-$C_4$-Alkoxygruppe oder mehrere $C_1$-$C_4$-Alkoxygruppen bzw. eine Halogengruppe oder mehrere Halogengruppen. Die $C_1$-$C_4$-Alkoxyreste können ebenso wie die bereits genannten $C_1$-$C_4$-Alkylreste von $R^5$ gerad- und verzweigtkettig sein und entsprechen den genannten einzelnen Resten. Als Halogen kommen F, Cl und Br in Betracht.

Die Salze der erfindungsgemäßen Verbindungen der Formel I sind Säureadditionssalze und leiten sich von physiologisch unbedenklichen Säuren ab. Solche physiologisch unbedenklichen Säuren sind anorganische Säuren, wie beispielsweise Chlorwasserstoffsäure, Salpetersäure, Phosphorsäure, Schwefelsäure, Bromwasserstoffsäure oder phosphorige Säure, oder organische Säuren wie beispielsweise aliphatische Mono- oder Dicarbonsäuren, phenylsubstituierte Alkancarbonsäuren, Hydroxyalkancarbonsäuren oder Alkandicarbonsäuren, aromatische Säuren oder aliphatische oder aromatische Sulfonsäuren.

Physiologisch unbedenkliche Salze dieser Säuren sind daher zum Beispiel das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Acetat, Propionat, Decanoat, Caprylat, Formiat, Isobutyrat, Caproat, Heptanoat, Propiolat, Malonat, Succinat, Suberat, Sebacat, Fumarat, Maleat, Mandelat, Butin-1,4-dioat, Hexin-1,4-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, β-Hydroxybutyrat, Glycollat, Malat, Tartrat, Methansulfonat, Propansulfonat, Naphthalin1-sulfonat oder Naphthalin-2-sulfonat.

Die Messungen von Blutdruck und Herzfrequenz wurden an der spontan hypertensiven Ratte und am narkotisierten, normotensiven Hund durchgeführt (siehe Tabelle 1 und 2). Wie ersichtlich, ruft MDL 19477 A eine Blutdrucksenkung hervor, die von einer ausgeprägten Herzfrequenzsteigerung begleitet wird. Bei der Verbindung nach Beispiel 1 tritt dagegen bei einer Dosierung, die eine ähnliche Blutdrucksenkung bewirkt, eine Herzfrequenzerniedrigung auf (SH-Ratte), oder es wird praktisch keine Herzfrequenzänderung beobachtet (Hund).

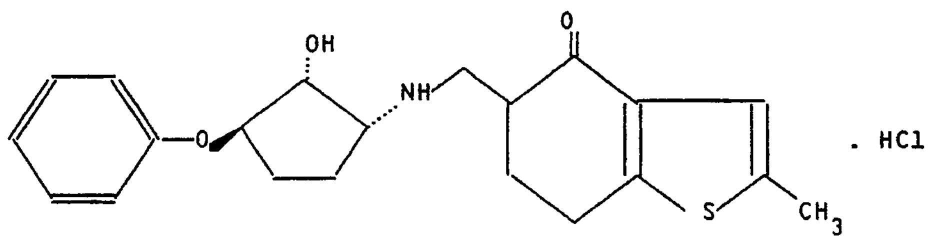

MDL 19744 A

Tabelle 1

| Blutdruck- und Herzfrequenzbeeinflussung an wachen, spontan hypertensiven Ratten[a] | | | |
|---|---|---|---|
| Verbindung | Dosis | PM[b] | HF[b] |
| MDL 19744 A | 1.0 mg/kg[c] | - 28% | + 20% |
| Beispiel 1 | 3.0 mg/kg[c] | - 36% | - 18% |

a) 3 Tiere pro Messung

b) PM = mittlerer arterieller Blutdruck, gemessen über einen Katheter in der rechten Carotisarterie; HF = Herzfrequenz. Es sind die Abweichungen in % gegenüber dem Ausgangswert, gemessen 25 Minuten nach Substanzapplikation, angege en.

c) i.v.-Applikation

Tabelle 2

| Blutdruck und Herzfrequenzbeeinflussung an narkotisierten, normtensiven Hunden[a)] | | | |
|---|---|---|---|
| Verbindung | Dosis | PM[b)] | HF[b)] |
| MDL 19744 A | 6.0 mg/kg[c)] | - 46 mmHg | + 65 $min^{-1}$ |
| Beispiel 1 | 3.0 mg/kg[d)] | - 33 mmHg | - 7 $min^{-1}$ |

a) Weibliche Beagle-Hunde mit Nembutal narkotisiert, MDL 19744 A wurde an 4 Tieren, Beispiel 1 an 2 Tieren unterrsucht.
b) PM = mittlerer arterieller Blutdruck, gemessen über einen Katheter in der linken Femoralarterie; HF = Herzfrequenz. Es sind die maximalen Abweichungen gegenüber einer Volumenkontrolle während oder nach der Substanzapplikation Uber einen Beobachtungszeitraum von insgesamt 90 Minuten angegeben.
c) Dauerinfusion über 20 Minuten
d) Dauerinfusion über 10 Minuten

Zur Verwendung der erfindungsgemäßen Verbindungen als Arzneimittel werden diese nach an sich bekannten Methoden der Galenik in die Form eines pharmazeutischen Präparats gebracht, die neben dem Wirkstoff insbesondere für die enterale Applikation geeignete organische oder anorganische inerte Trägermaterialien, wie zum Beispiel Wasser, Gelatine, Gummi arabicum, Milchzucker, Stärke, Magnesiumstearat, Talk, Polyalkylenglykole usw. enthalten können. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs-, Stabilisierungs-, Netzmittel oder Emulgatoren, Salze zur Veränderung des osmotischen Drucks oder Puffer.

Für die Verwendung am Menschen beträgt die Dosierung 50 bis 1000 mg pro Tag.

Die Erfindung betrifft auch ein Verfahren zur Herstellung von 2-Hydroxymethylthiophen-Derivaten der Formel I, das dadurch gekennzeichnet ist, daß man 2-Hydroxymethyl-4-propenoyl-thiophen-Derivate der Formel II

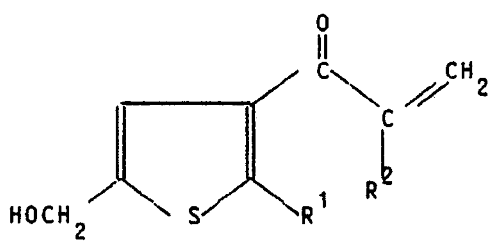

(II),

worin
$R^1$ und $R^2$ die oben genannte Bedeutung haben, mit einem Amin der Formel III

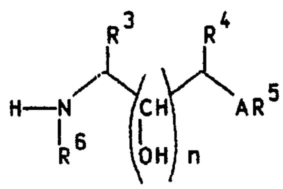

(III),

worin n, A, $R^3$, $R^4$, $R^5$ und $R^6$ die oben angegebenen Bedeutungen haben, umsetzt und gewünschtenfalls anschließend mit anorganischen oder organischen Säuren umsetzt.

Die Herstellung der Verbindungen der allgemeinen Formel I erfolgt nach an sich bekannten Verfahren.

Als geeignete Amine seien Piperidinderivate, Phenylethylaminderivate oder Amine der allgemeinen Formel III mit n = 1 genannt.

Die Reaktion wird bei Temperaturen bis zu 50°C durchgeführt und ist im allgemeinen nach ca. 20 Stunden beendet.

Als Lösungsmittel sind niedere Alkohole wie beispielsweise Methanol und Ethanol geeignet.

Die Herstellung der Ausgangsverbindungen ist bekannt oder erfolgt nach bekannten Methoden.

Die nachfolgenden Beispiele sollen das erfindungsgemäße Verfahren erläutern.

## Darstellung der Ausgangsverbindungenen

4,5,6,7-Tetrahydro-2-hydroxymethyl-5-methylen-benzothiophen-4-on

a) 50,0 g 4,5,6,7-Tetrahydro-benzothiophen-4-on-2-carbonsäure [Chem. Ber. 99, 3309 (1966)] werdem mit 200 ml Methanol und 10 ml Schwefelsäure 10 h am Rückfluß erhitzt. Nach dem Abkühlen wird das Reaktionsgemisch in 2 l Wasser eingerührt und der Niederschlag wird abgesaugt. Das Rohprodukt wird in Essigester gelöst und mit gesättigter Natriumhydrogencarbonatlösung und Wasser gewaschen. Die organische Phase wird abgetrennt, über Natriumsulfat getrocknet und nach dem Abfiltrieren wird das Lösungsmittel entfernt. Es werden 37,9 g 4,5,6,7-Tetrahydrobenzothiophen-4-on-2-carbonsäuremethylester vom Schmelzpunkt 93 - 94 °C erhalten.

b) 37,0 g der unter a) erhaltenen Verbindung werden mit 20 ml Ethylenglykol und 3,4 g p-Toluolsulfonsäure in 500 ml Toluol gelöst und am Wasserabscheider 20 h erhitzt. Nach dem Abkühlen wird das Reaktionsgemisch mit Natriumhydrogencarbonatlösung gewaschen. Die organische Phase wird abgetrennt, über Natriumsulfat getrocknet und nach dem Abfiltrieren eingeengt. Das Rohprodukt wird mit einem Hexan-Essigester-Gemisch (8:2) an Kieselgel chromatographiert. Es werden 26,3 g 4,4-Ethylendioxy-4,5,6,7-tetrahydrobenzothiophen-2-carbonsäuremethylester als Öl erhalten.

c) Eine Lösung aus 16,2 g der unter b) erhaltenen Verbindung in 50 ml Tetrahydrofuran wird unter Kühlung zu einer Suspension von 4,8 g Lithiumaluminiumhydrid in 150 ml Tetrahydrofuran getropft. Nach 2 h wird der Ansatz vorsichtig mit Natriumcarbonatlösung versetzt. Der Niederschlag wird abfiltriert und mit Essigester nachgewaschen. Die organische Phase wird abgetrennt, eingeengt und anschließend in Dioxan aufgenommen und mit 20 ml zweinormaler Salzsäure versetzt. Nach 1 h bei Raumtemperatur wird mit Natriumcarbonat neutralisiert und eingeengt. Der Rückstand wird mit Dichlormethan ausgerührt, es wird abfiltriert und die Lösung wird über Natriumsulfat getrocknet. Nach dem Abfiltrieren und Einengen werden 10,9 g 4,5,6,7-Tetrahydro-2-hydroxymethyl-benzothiophen-4-on vom Schmelzpunkt 53 - 54 °C erhalten.

d) Eine Mischung aus 41,8 g der unter c) erhaltenen Verbindung, 68,9 g Paraformaldehyd und 177,5 g N-Methylaniliniumtrifluoracetat wird in 600 ml Tetrahydrofuran 18 h am Rückfluß erhitzt. Nach dem Abkühlen wird das Reaktionsgemisch in 4 l Diethylether eingerührt. Der Niederschlag wird abfiltriert und das Filtrat wird mit Natriumhydrogencarbonatlösung und Wasser gewaschen. Die organische Phase wird abgetrennt, über Natriumsulfat getrocknet und nach dem Abfiltrieren eingeengt. Das Rohprodukt wird mit einem Cyclohexan-Essigester-Gemisch (6:4) an Kieselgel chromatographiert. Es werden 16,2 g 4,5,6,7-Tetrahydro-2-hydroxymethyl-5-methylen-benzothiophen-4-on vom Schmelzpunkt 41 - 43 °C erhalten.

(+)-Z-2-Hydroxy-E-3-phenoxy-cyclopentylamin

19,3 g racemisches Z-2-Hydroxy-(E)-3-phenoxy-cyclopentylamin [J.R. McCarthy et al., J. Med. Chem. 28, 1142 (1985)] und 15,2 g L-(+)-Mandelsäure werden in 200 ml Ethanol in der Wärme gelöst. Das beim Abkühlen auskristallisierende Salz wird noch zweimal aus Ethanol umkristallisiert. Es werden 8,3 g (24 % d.Th.) des Salzes mit Schmp. 190 - 199 °C und $[\alpha]_D$ +59,2 °C (c = 0,4, $CH_3OH$) erhalten. Bei Freisetzung der Base aus dem Salz mit 2n NaOH werden 3,7 g der Verbindung mit Schmp. 66-68 °C (aus Wasser) und $[\alpha]_D$ +43,2 °C (c = 1,0, $CH_3OH$) erhalten.

(-)-Z-2-Hydroxy-(E)-3-phenoxy-cyclopentylamin

5,8 g racemisches Z-2-Hydroxy-(E)-3-phenoxy-cyclopentylamin und 4,56 g (D-(-)-Mandelsäure werden wie vorstehend umgesetzt und umkristallisiert. Es werden 3,2 g (31 % d.Th.) des Salzes mit Schmp. 193 -

197 °C und $[\alpha]_D$ -59,3 °C (c = 0,4, $CH_3OH$) erhalten. Aus diesem Salz wird mit 2n NaOH das Amin freigesetzt, wobei 1,4 g der Verbindung mit Schmp. 66 - 68 °C (aus Wasser) und $[\alpha]_D$ -42,9 °C (c = 1,0, $CH_3OH$) erhalten werden.

2-Hydroxymethyl-4-propenoyl-thiophen

a) 1,00 g 4-Bromthiophen-2-aldehyd wird in 10 ml 2-Propanol gelöst und mit 0,60 g Natriumborhydrid versetzt. Nach 2 Stunden wird das Reaktionsgemisch mit verdünnter Salzsäure versetzt und mit Essigester extrahiert. Die organische Phase wird über Natriumsulfat getrocknet und nach dem Abfiltrieren das Lösungsmittel entfernt. Es wurden 0,98 g 4-Brom-2-hydroxymethyl-thiophen als Öl erhalten.

b) Eine Lösung von 13,5 g der unter a) erhaltenen Verbindung, 27,8 g 1-Ethoxyvinyltributylzinn und 2,5 g 1,1-Bis-diphenylphosphinoferrocen-Palladium-II-chlorid (J.Am.Chem.Soc. 106, 158 (1984)) in 80 ml Toluol wird für 12 Stunden auf 100 °C erhitzt. Nach dem Abkühlen wird das Reaktionsgemisch in Essigester aufgenommen und mit Wasser gewaschen. Die organische Phase wird abgetrennt, über Natriumsulfat getrocknet und nach dem Abfiltrieren das Lösungsmittel entfernt. Der Rückstand wird mit einem Hexan-Essigester-Gemisch (8:2) chromatographiert. Es werden 5,4 g 4-Acetyl-2-hydroxymethyl-thiophen als Öl erhalten.

c) Eine Mischung aus 2,20 g der unter b) erhaltenen Verbindung, 4,23 g Paraformaldehyd und 10,00 g N-Methylaniliniumtrifluoracetat wird in 50 ml Tetrahydrofuran 12 Stunden am Rückfluß erhitzt. Nach dem Abkühlen wird das Reaktionsgemisch in 250 ml Diethylether eingerührt. Der Niederschlag wird abfiltriert und das Filtrat wird mit Natriumhydrogencarbonatlösung und Wasser gewaschen. Die organische Phase wird abgetrennt, über Natriumsulfat getrocknet und nach dem Abfiltrieren eingeengt. Das Rohprodukt wird mit einem Cyclohexan-Essigester-Gemisch (6:4) an Kieselgel chromatographiert. Es werden 0,85 g 2-Hydroxymethyl-4-propenoyl-thiophen als Öl erhalten.

Beispiel 1

5-[N-((Z)-2-Hydroxy-(E)-3-phenoxy-cyclopentyl)-aminomethyl]-4,5,6,7-tetrahydro-2-hydroxymethyl-benzothiophen-4-on, Hydrochlorid

1,34 g 4,5,6,7-Tetrahydro-2-hydroxymethyl-5-methylen-benzothiophen-4-on und 1,30 g (Z)-2-Hydroxy-(E)-3-phenoxy-cyclopentylamin werden in 10 ml Ethanol bei Raumtemperatur 20 h gerührt. Das Reaktionsgemisch wird mit etherischem Chlorwasserstoff versetzt und eingeengt. Der Rückstand wird aus 2-Propanol umkristallisiert. Es werden 0,45 g 5-[N-(Z)-2-Hydroxy-(E)-3-phenoxy-cyclopentyl)-aminomethyl]-4,5,6,7-tetrahydro-2-hydroxymethyl-benzothiophen-4-on, Hydrochlorid, vom Schmelzpunkt 159 - 161 °C erhalten.

Beispiel 2

4,5,6,7-Tetrahydro-2-hydroxymethyl-5-(2-hydroxy-3-phenoxy-propylaminomethyl)-benzothiophen-4-on, Hydrochlorid

1,00 g 4,5,6,7-Tetrahydro-2-hydroxymethyl-5-methylen-benzothiophen-4-on und 0,86 g 2-Hydroxy-3-phenoxy-propylamin werden in 10 ml Ethanol bei Raumtemperatur 20 h gerührt. Das ausgefallene Produkt wird abgesaugt, in 10 ml Methanol aufgenommen, mit Salzsäure versetzt und eingeengt. Der Rückstand wird aus 2-Propanol umkristallisiert. Es werden 0,61 g 4,5,6,7-Tetrahydro2-hydroxymethyl-5-(2-hydroxy-3-phenoxy-propylaminomethyl)-benzothiophen-4-on, Hydrochlorid, vom Schmelzpunkt 128 - 132 °C erhalten.

Beispiel 3

5-[N-2-(3,4-Dimethoxyphenyl)-ethyl]-aminomethyl]-4,5,6,7-tetrahydro-2-hydroxymethyl-benzothiophen-4-on, Hydrochlorid

1,00 g 4,5,6,7-Tetrahydro-2-hydroxymethyl-5-methylen-benzothiophen-4-on und 0,87 ml 2-(3,4-Dimethoxyphenyl)-ethylamin werden in 10 ml Ethanol bei Raumtemperatur 20 h gerührt. Das Reaktionsgemisch wird mit etherischem Chlorwasserstoff versetzt und eingeengt. Der Rückstand wird aus 2-Propanol umkristallisiert. Es werden 0,85 g 5-[N-2-(3,4-Dimethoxyphenyl)-ethyl]-aminomethyl]-4,5,6,7-tetrahydro-2-hydroxy methyl-benzothiophen-4-on, Hydrochlorid vom Schmelzpunkt 158 - 162 °C erhalten.

Beispiel 4

5-[N-[(Z)-2-Hydroxy-(E)-3-(2,4-difluorphenoxy)-cyclopentyl]-aminomethyl]-4,5,6, 7-tetrahydro-2-hydroxymethyl-benzothiophen-4-on, Hydrochlorid

0,90 g 4,5,6,7-Tetrahydro-2-hydroxymethyl-5-methylen-benzothiophen-4-on und 1,06 g (Z)-2-Hydroxy-(E)-3-(2,4-difluorphenoxy)-cyclopentylamin werden in 10 ml Ethanol bei Raumtemperatur 20 h gerührt. Das ausgefallene Produkt wird abgesaugt, in 10 ml Methanol aufgenommen, mit Salzsäure versetzt und eingeengt. Der Rückstand wird aus 2-Propanol umkristallisiert. Es werden 0,74 g 5-[N-[(Z)-2-Hydroxy-(E)-3-(2,4-difluorphenoxy)-cyclopentyl]-aminomethyl]-4,5,6,7-tetrahydro-2-hydroxymethyl-benzothiophen-4-on, Hydrochlorid vom Schmelzpunkt 156 -158 °C erhalten.

Beispiel 5

5-[N-[(Z)-2-Hydroxy-(E)-3-(4-hydroxyphenoxy)-cyclopentyl]-aminomethyl]-4,5,6,7-tetrahydro-2-hydroxymethyl-benzothiophen-4-on, Hydrochlorid

1,00 g 4,5,6,7-Tetrahydro-2-hydroxymethyl-5-methylen-benzothiophen-4-on und 1,08 g (Z)-2-Hydroxy-(E)-3-(4-hydroxyphenoxy)-cyclopentylamin werden 10 ml Ethanol bei Raumtemperatur 20 h gerührt. Das ausgefallene Produkt wird abgesaugt, in 10 ml Methanol aufgenommen, mit Salzsäure versetzt und eingeengt. Der Rückstand wird aus 2-Propanol umkristallisiert. Es werden 0,85 g 5-[N-[(Z)-2-Hydroxy-(E)-3-(4-hydroxyphenoxy)-cyclopentyl]-aminomethyl]-4,5,6,7-tetrahydro-2-hydroxymethyl-benzothiophen-4-on, Hydrochlorid vom Schmelzpunkt 165 -168 °C erhalten.

Beispiel 6

5-[N-(2-Phenyl-ethyl)-aminomethyl]-4,5,6,7-tetrahydro-2-hydroxymethyl-benzothiophen-4-on, Hydrochlorid

1,00 g 4,5,6,7-Tetrahydro-2-hydroxymethyl-5-methylen-benzothiophen-4-on und 0,65 ml 2-Phenyl-ethylamin werden in 10 ml Ethanol bei Raumtemperatur 20 h gerührt. Das Reaktionsgemisch wird mit etherischem Chlorwasserstoff versetzt und eingeengt. Der Rückstand wird aus 2-Propanol umkristallisiert. Es werden 0,13 g 5-[N-(2-Phenyl-ethyl)-aminomethyl]-4,5,6,7-tetrahydro-2-hydroxymethyl-benzothiophen-4-on, Hydrochlorid vom Schmelzpunkt 127 - 129 °C erhalten.

Beispiel 7

5-[(4-Methylpiperidino)-methyl]-4,5,6,7-tetrahydro-2-hydroxymethyl-benzothiophen-4-on, Hydrochlorid

1,00 g 4,5,6,7-Tetrahydro-2-hydroxymethyl-5-methylen-benzothiophen-4-on und 0,61 ml 4-Methylpiperidin werden in 5 ml Ethanol bei Raumtemperatur 20 h gerührt. Das Reaktionsgemisch wird eingeengt und mit einem Dichlormethan-Methanol-Gemisch (8:2) an Kieselgel chromatographiert. Das Rohprodukt wird in Ethanol gelöst, mit Salzsäure versetzt und eingeengt. Der Rückstand wird aus 2-Propanol umkristallisiert. Es werden 0,69 g 5-[(4-Methylpiperidino)-methyl]-4,5,6,7-tetrahydro-2-hydroxymethyl-benzothiophen-4-on, Hydrochlorid vom Schmelzpunkt 171 - 173 °C erhalten.

Beispiel 8

(+)-5-[N-((Z)-2-Hydroxy-(E)-3-phenoxy-cyclopentyl)-aminomethyl]-4,5,6,7-tetrahydro-2-hydroxymethyl-benzothiophen-4-on, Hydrochlorid

1,26 g 4,5,6,7-Tetrahydro-2-hydroxymethyl-5-methylen-benzothiophen-4-on und 1,50 g (+)-(Z)-2-Hydroxy-(E)-3-phenoxy-cyclopentylamin werden in 10 ml Ethanol bei Raumtemperatur 20 h gerührt. Das Reaktionsgemisch wird mit etherischem Chlorwasserstoff versetzt und eingeengt. Der Rückstand wird aus 2-Propanol umkristallisiert. Es werden 0,85 g (+)-5-[N-((Z)-2-Hydroxy-(E)-3-phenoxy-cyclopentyl)-aminomethyl]-4,5,6,7-tetrahydro-2-hydroxymethyl-benzothiophen-4-on, Hydrochlorid vom Schmelzpunkt 125 - 128 °C erhalten.

Beispiel 9

(-)-5-[N-((Z)-2-Hydroxy-(E)-3-phenoxy-cyclopentyl)-aminomethyl]-4,5,6,7-tetrahydro-2-hydroxymethyl-benzothiophen-4-on, Hydrochlorid

1,00 g 4,5,6,7-Tetrahydro-2-hydroxymethyl-5-methylen-benzothiophen-4-on und 1,20 g (-)-(Z)-2-Hydroxy-(E)-3-phenoxy-cyclopentylamin werden in 10 ml Ethanol bei Raumtemperatur 20 h gerührt. Das Reaktionsgemisch wird mit etherischen Chlorwasserstoff versetzt und eingeengt. Der Rückstand wird aus 2-Propanol umkristallisiert. Es werden 0,85 g (-)-5-[N-((Z)-2-Hydroxy-(E)-3-phenoxy-cyclopentyl)-aminomethyl]-4,5,6,7-tetrahydro-2-hydroxymethyl-benzothiophen-4-on, Hydrochlorid vom Schmelzpunkt 123 - 127 °C erhalten.

Beispiel 10

4-[N-((Z)-2-Hydroxy-(E)-3-phenoxy-cyclopentyl)-aminopropionyl]-2-hydroxymethylthiophen, Hydrogenfumarat

275 mg 2-Hydroxymethyl-4-propenoyl-thiophen und 316 mg Z-2-Hydroxy-E-phenoxy-cyclopentylamin werden in 5 ml Ethanol bei Raumtemperatur 47 h gerührt. Das Reaktionsgemisch wird eingeengt und mit einem Dichlormethan-Methanol-Gemisch (95:5) an Kieselgel chromatographiert. Das Rohprodukt wird in Ethanol gelöst, mit Fumarsäure versetzt und eingeengt. Der Rückstand wird aus 2-Propanol umkristallisiert. Es werden 204 mg 4-[N-((Z)-2-Hydroxy-(E)-3-phenoxy-cyclopentyl)-aminopropionyl]-2-hydroxymethyl-thiophen, Hydrogenfumarat erhalten.

**Ansprüche**

1. 2-Hydroxymethylthiophen-Derivate der Formel I

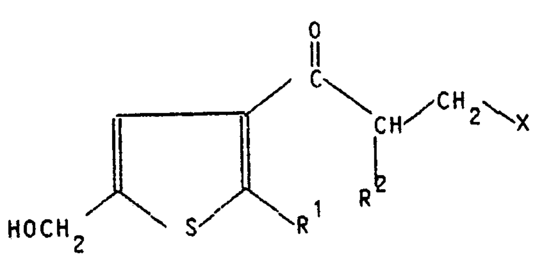

(I),

worin
$R^1$ und $R^2$ Wasserstoff oder gemeinsam eine Ethylengruppe,
$R^3$ und $R^4$ Wasserstoff oder gemeinsam eine Ethylengruppe und

X die Gruppe

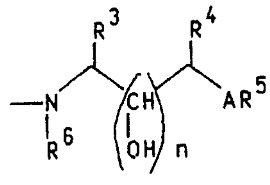

mit $R^5$ in der Bedeutung eines $C_1$-$C_4$-Alkyls oder eines gegebenenfalls substituierten Phenylrestes, mit n in der Bedeutung 1 oder 0, mit A in der Bedeutung eines Sauerstoffatoms oder einer direkten Bindung oder, wenn n = 0 und $R^3$ und $R^4$ = H sind, mit einer gemeinsamen $-CH_2-CH_2-CH-$ -Gruppe für $R^6$ und A darstellen sowie deren Säureadditionssalze.

2. 5-[N-((Z)-2-Hydroxy-(E)-3-phenoxy-cyclopentyl)-aminomethyl]-4,5,6,7-tetrahydro-2-hydroxymethylbenzthiophen-4-on, Hydrochlorid.

3. Verfahren zur Herstellung von 2-Hydroxymethylthiophen-Derivaten der Formel I, das dadurch gekennzeichnet ist, daß man 2-Hydroxymethyl-4-propenoyl-thiophen-Derivate der Formel II

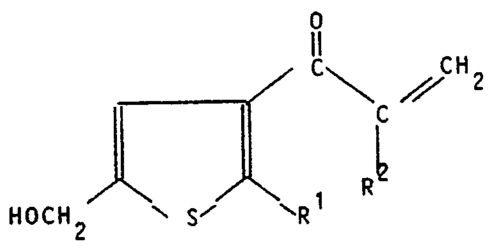

(II),

worin

$R^1$ und $R^2$ die oben genannte Bedeutung haben, mit einem Amin der Formel III

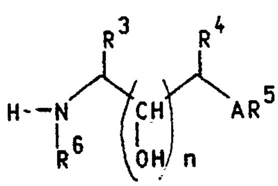

(III)

worin n, A, $R^3$, $R^4$, $R^5$ und $R^6$ die oben angegebenen Bedeutungen haben, umsetzt und gewünschtenfalls anschließend mit anorganischen oder organischen Säuren umsetzt.

4. Arzneimittel, bestehend aus einer oder mehreren Verbindungen der Formel I und üblichen Hilfs- oder Trägerstoffen.

Europäisches Patentamt

# EUROPÄISCHER RECHERCHENBERICHT

Nummer der Anmeldung

EP 89250096.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2 - 0 127 143 (MERRELL DOW) * Ansprüche 1-5, 17-20 * -- | 1,3,4 | C 07 D 333/20<br>C 07 D 333/54<br>A 61 K 31/38 |
| A | DE - A - 1 670 547 (DEGUSSA) * Seiten 1-4, Beispiele 1,9 * ---- | 1,3,4 | |
| | | | RECHERCHIERTE SACHGEBIETE (Int. Cl.4) |
| | | | C 07 D 333/00<br>A 61 K 31/00 |

Der vorliegende Recherchenbericht wurde für alle Patentansprüche erstellt.

| Recherchenort | Abschlußdatum der Recherche | Prüfer |
|---|---|---|
| WIEN | 23-01-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82